(19) Europäisches Patentamt
European Patent Office
Office européen des brevets

(11) Publication number: 0 138 468 B1

(12) # EUROPEAN PATENT SPECIFICATION

(45) Date of publication of patent specification: 05.04.89

(51) Int. Cl.$^4$: C 07 D 303/40, C 07 B 57/00

(21) Application number: 84306582.2

(22) Date of filing: 27.09.84

(54) **Method for preparing optically active dialkyl trans-epoxysuccinates.**

(30) Priority: 19.10.83 JP 195695/83

(43) Date of publication of application: 24.04.85 Bulletin 85/17

(45) Publication of the grant of the patent: 05.04.89 Bulletin 89/14

(84) Designated Contracting States: DE FR GB IT

(56) References cited:
DE-A-3 212 882
CHEMICAL ABSTRACTS, vol. 96, no. 9, March 1, 1982, Columbus, Ohio, USA TAISHO PHARMACEUTICAL CO., LTD. "Optically active diethyl epoxysuccinates" page 593, column 2, abstract-no. 68 794z
CHEMICAL ABSTRACTS, vol. 97, no. 23, December 6, 1982, Columbus, Ohio, USA SAIGO, KAZUHIKO; OGAWA, SHIGEO; KIKUCHI, SHIGETOSHI; KASAHARA, ATSUSHI; NOHIRA, HIROYUKI "Optical resolution of 2-amino-1,2-diphenyl-ethanol by preferential crystallization and its utilization in fractional crystallization and enentioselective reduction of prochiral ketones" page 549, column 2, abstract-no. 197 904e

(73) Proprietor: TAISHO PHARMACEUTICAL CO. LTD
24-1 Takata 3-chome Toshima-ku
Tokyo 171 (JP)

(72) Inventor: Hatayama, Katsuo
35-3, Horisakichodanchi 1200-215, Horisakicho
Omiya-Shi (JP)
Inventor: Yokoo, Chihiro
8-5, Mochida-4-chome
Gyoda-Shi (JP)
Inventor: Murata, Mitsuo
2-15-202, Washinomiyadanchi Washinomiyacho
Kitakatsushika-Gun Saitama-Ken (JP)
Inventor: Sota, Kaoru
1158-11, Shimotomi
Tokorozawa-Shi (JP)

(74) Representative: Paget, Hugh Charles Edward et al
MEWBURN ELLIS & CO. 2/3 Cursitor Street
London EC4A 1BQ (GB)

(56) References cited:
CHEMICAL ABSTRACTS, vol. 87, no. 11, September 12, 1977, Columbus Ohio USA SEKI TERUYA "N-(3-trans-Ethoxycarbonyloxirane-2-carbonyl)leucylagmatine" page 627, column 1, abstract-no. 85 238c

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 138 468 B1

## Description

This invention relates to a method for preparing optically active dialkyl trans-epoxysuccinates.

It is known that optically active isomers of dialkyl epoxysuccinates can be obtained by dehydrobromination of particular isomers of dialkyl bromosuccinates (Chem. Abs. (1982), *96*, 593, No. 68794z, of JP—A—81—110683) and that the free DL-trans epoxysuccinic acid can be resolved into the D- or L-form using D- or L-erythro-2-amino-1,2-diphenylethanol (Saigo et al, Bull Chem. Soc. Japan (1982), 55(5), 1568—73; Chem. Abs. (1982), *97*, 197904e).

The following methods for preparing optically active dialkyl trans-epoxysuccinates are also known:

(1) a method which comprises esterifying D-transepoxysuccinic acid (which is obtained by fermentation) or its barium salt, with a lower alcohol in the presence of sulfuric acid [Max W. Miller; Journal of Organic Chemistry (1963), *28*, 1148, and

(2) a method for preparing a diethyl D- (or L-)trans-epoxysuccinate which comprises resolving DL-trans-epoxysuccinic acid (obtained by epoxidation of fumaric acid), with (+) or (−) (1-naphthyl)ethylamine, removing the amine, and then esterifying the resulting D- or L-trans-epoxysuccinic acid with ethanol in the presence of sulfuric acid (Nohira et al, "The Previous Substances II of the Lectures at the 48th National Meeting of the Chemical Society of Japan" (1983), item 3008 page 831).

However, by the fermentation method indicated in the above method (1), only the dialkyl D-trans-epoxysuccinate can be obtained. On the other hand, the method involving resolution indicated in the above method (2) has drawbacks such as the necessity for the complicated steps of preparing the free acid after resolution thereof and before esterification to obtain the diethyl D- or L-trans-epoxysuccinate.

We have now succeeded in preparing an optically active dialkyl trans-epoxysuccinate more easily and in better yield by reacting DL-trans-epoxysuccinic acid with an optically active arginine to form a diastereoisomer salt and then esterifying the salt with a lower alcohol in the presence of an acid catalyst. By this method, the acid-amine salt is esterified directly, i.e. without the necessity for isolating the optically active free trans-epoxysuccinic acid.

Thus, the present invention provides a method for preparing an optically active dialkyl trans-epoxysuccinate of the formula (I)

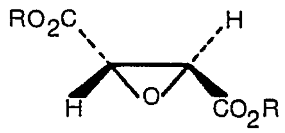

(I)

wherein R is a $C_{1-3}$ alkyl group, which method includes a reaction step in which DL-trans epoxysuccinic acid is reacted with a D- or L-amino compound, and an esterification step carried out in the presence of an acid catalyst and using an alcohol of the formula ROH, wherein R is as defined above, characterised in that, in combination,

(a) in the reaction step, the amino compound is a D- or L-arginine, whereby a diastereoisomer trans-epoxysuccinic acid/arginine salt is formed,

(b) the said esterification step is carried out directly on the said diastereoisomer trans-epoxysuccinic acid/arginine salt to form the said optically active dialkyl trans-epoxysuccinate, and

(c) in the esterification step, the acid catalyst is selected from sulphuric, hydrochloric and p-toluenesulfonic acids, which acid catalyst is present in an amount of from 1.1 to 3 moles inclusive per mole of the said diastereoisomer salt.

The esterification may be carried out at a temperature ranging from 20 to 100°C, preferably 40 to 100°C, for 1 to 24 hours, preferably 4 to 7 hours.

The alcohol of formula ROH having 1, 2 or 3 carbon atoms is methyl, ethyl, n-propyl or i-propyl alcohol, among which methyl and ethyl alcohols are preferred.

The diasteromer salt, used as starting material for subsequent esterification may be prepared, for example, as follows: DL-trans-epoxysuccinic acid is dissolved in methanol while warming to 40 to 60°C. To the resulting solution is added an aqueous solution of D-arginine or L-arginine with stirring, the mixture is allowed to stand at room temperature overnight, and the resulting crystals are collected by filtration to give D-trans-epoxysuccinic acid·D-arginine salt or L-trans-epoxysuccnic acid·L-arginine salt. On the other hand, the filtrate is treated in a conventional manner to give L-trans-epoxysuccnic acid D-arginine salt or D-trans-epoxysuccinic acid L-arginine salt.

The desired compound of formula (I) is prepared as follows: The diastereomer salt is suspended in the alcohol which corresponds to the desired ester moiety of the end product, and the suspension is heated in the presence of the acid catalyst for esterification to give the compound of formula (I), which is then purified according to a conventional manner.

The compounds of formula (I) are useful as intermediates in processes for the production of ethyl (2S, 3S)-3-[(S)-3-methyl-1-(3-methyl-butylcarbamoyl) butylcarbamoyl]-oxirane-2-carboxylate and compounds related thereto and in synthetic processes for producing various products having physiological activity (see US—A—4393228), which were previously obtained from natural sources.

The method of the present invention makes it possible to obtain an optically active dialkyl trans-epoxysuccinate easily in better yield without the step of isolating the optically active trans-epoxysuccinic acid, and therefore, the method is very useful as an industrial method for producing intermediates for medicines.

The present invention will be more concretely illustrated below by the following examples

Example 1

(1) To a solution of 66.05 g of DL-trans-epoxysuccinic acid dissolved in 1000 ml of methanol was added a solution of 87.1 g of L-arginine dissolved in 250 ml of water while warming to 50°C with stirring. The mixture was allowed to stand at room temperature overnight. The crystals formed were collected by filtration, and washed with a small amount of methanol to give 73.1 g (yield: 95.5%) of L-trans epoxysuccinic acid·L-arginine salt.

m.p. 181—183°C
$[\alpha]_D^{26} = +51.6°$ ($C = 1.02$, $H_2$)

(2) In 150 ml of ethanol was suspended 15.3 g of L-trans-epoxysuccinic acid·L-arginine salt obtained above. To the suspension was added gradually 14.7 g of conc. sulfuric acid under ice-cooling with stirring, and the mixture was heated at 60°C with stirring for 4 hours.

Subsequently, the excess ethanol was removed by evaporation under reduced pressure. To the resulting residue was added 150 ml of ice water, and the mixture was extracted with ethyl acetate. The ethyl acetate layer was washed, in turn, with water and a saturated aqueous sodium chloride solution, dried over magnesium sulfate, and distilled to give 7.2 g of diethyl L-trans-epoxysuccinate.

b.p. 80°C/0.9 mmHg
$[\alpha]_D^{26} = +110.5°$ ($C = 1.12$, $C_2H_5OH$)

Example 2

(1) To a solution of 66.05 g of DL-trans-epoxysuccinic acid dissolved in 1000 ml of methanol was added a solution of 87.1 g of D-arginine dissolved in 250 ml of water while warming to 50°C with stirring. The mixture was allowed to stand at room temperature overnight. The crystals formed were collected by filtration, and recrystallized from a mixture of methanol and water (2:1 by volume) to give 7.1 g of D-trans-epoxysuccinic acid·D-arginine salt as needles.

m.p. 172°C (decomposition)
$[\alpha]_D^{26} = -54.0°$ ($C = 1.00$, $H_2O$)

(2) To a suspension of 17.5 g of D-trans-epoxysuccinic acid·D-arginine salt in 160 ml of ethanol was added 16.8 g of conc. sulfuric acid, and subsequently the mixture was treated by the method similar to that of Example 1 to give 7.6 g of diethyl D-trans-epoxysuccinate.

b.p. 85°C/1.0 mmHg
$[\alpha]_D^{26} = -109.5°$ ($C = 1.11$, $C_2H_5OH$)

Example 3

To a suspension of 15 g of D-trans-epoxysuccinic acid·D-arginine salt dissolved in 150 ml of methanol was added 14.4 g of conc. sulfuric acid and subsequently the mixture was treated by a method similar to that of Example 1 to give 5.1 g of dimethyl D-trans-epoxysuccinate.

m.p. 74 — 75°C
$[\alpha]_D^{26} = -125°$ ($C = 1.00$, $C_2H_5OH$)

**Claims**

1. A method for preparing an optically active dialkyl trans-epoxysuccinate of the formula (I)

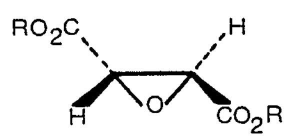

(I)

wherein R is a $C_{1-3}$ alkyl group, which method includes a reaction step in which DL-trans epoxysuccinic acid is reacted with a D- or L-amino compound, and an esterification step carried out in the presence of an acid catalyst and using an alcohol of the formula ROH, wherein R is as defined above, characterised in that, in combination,

(a) in the reaction step, the amino compound is a D- or L-arginine, whereby a diastereoisomer trans-epoxysuccinic acid/arginine salt is formed,

(b) the said esterification step is carried out directly on the said diastereoisomer trans-epoxysuccinic acid/arginine salt to form the said optically active dialkyl trans-epoxysuccinate, and

(c) in the esterification step, the acid catalyst is selected from sulphuric, hydrochloric and p-toluenesulfonic acids, which acid catalyst is present in an amount of from 1.1 to 3 moles inclusive per mole of the said diastereoisomer salt.

2. A method according to Claim 1, wherein the said alcohol is methyl alcohol, ethyl alcohol, n-propyl alcohol or i-propyl alcohol.

3. A method according to Claim 1 or Claim 2 wherein the esterification is carried out at a temperature ranging from 20 to 100°C for 1 to 24 hours.

**Patentansprüche**

1. Verfahren zur Herstellung eines optisch aktiven Dialky-trans-epoxysukzinaters der Formel (I)

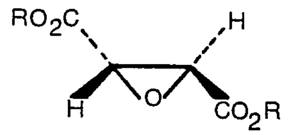

(I)

worin R eine $C_{1-3}$-Alkylgruppe bedeutet, wobei das Verfahren eine Reaktionsstufe einschließt, in welcher DL-trans-epoxybernsteinsäure mit einer D- oder L-Amino-Verbindung umgesetzt wird, und eine Veresterungsstufe in Gegenwart eines sauren Katalysators und unter Verwendungt eines Alkohols der Formel ROH, in welcher R die vorher angegebene Bedeutung hat, dadurch gekennzeichnet, daß in Kombination

(a) in der Reaktionsstufe die Aminosäure D- oder L-Arginin ist, und sich ein diastereoisomeres Trans-epoxybernsteinsäure/Argininsalz bildet,

(b) die Veresterungsstufe direkt mit dem diasterioisomeren Trans-epoxybernsteinsäure/Argininsalz durchgeführt wird unter Ausbildung des optisch aktiven Dialkyl-trans-epoxysukzinates, und

(c) in der Veresterungsstufe der Säurekatalysator ausgewählt ist aus Schwefelsäure, Salzsäure und para-Toluolsulfonsäure, wobei der Säurekatalysator in einer Menge von 1,1 bis 3 Molen inklusiv pro Mol des diastereoisomeren Salzes vorliegt.

2. Verfahren gemäß Anspruch 1, bei dem der Alkohol Methylalkohol, Ethylalkohol, n-Propylalkohol oder Isopropylalkohol ist.

3. Verfahren gemäß Anspruch 1 oder 2, bei dem die Veresterung in einem Temperaturbereich von 20 bis 100°C während 1 bis 24 Stunden durchgeführt wird.

**Revendications**

1. Procédé de préparation d'un trans-époxysuccinate de dialkyle optiquement actif de formule (I)

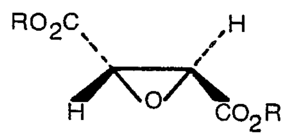

(I)

dans laquelle R est un groupement alkyle en $C_1$—$C_3$, procédé qui comporte une étape de réaction dans laquelle on fait réagir l'acide DL-trans-époxysuccinique avec un composé D- ou L-amino, et une étape d'estérification effectuée en présence d'un catalyseur acide et en utilisant un alcool de formule ROH dans laquelle R est tel que défini précédemment, caractérisé en ce que, en combinaison,

a) dans l'étape de réaction, le composé amino est une D- ou L-arginine, ce qui forme un sel acide trans-époxysuccinique/arginine diastéréoisomère,

b) ladite étape d'estérification est effectuée directement sur ledit sel acide trans-époxysuccinique/arginine diastéréoisomère pour former ledit trans-époxysuccinate de dialkyle optiquement actif, et

c) dans l'étape d'estérification, le catalyseur acide est choisi parmi les acides sulfurique, chlorhydrique et p-toluenesulfonique, le catalyseur acide étant présent à raison de 1,1 à 3 (compris) moles par mole dudit sel diastéréoisomère.

2. Procédé selon la revendication 1, dans lequel ledit alcool est l'alcool méthylique, l'acool éthylique, l'alcool n-propylique ou l'alcool isopropylique.

3. Procédé selon la revendication 1 ou 2, dans lequel l'estérification est faite à une température comprise entre 20 et 100°C pendant 1 à 24 heures.